# EUROPÄISCHE PATENTSCHRIFT

(11) **EP 0 618 447 B1**
(45) Veröffentlichungstag und Bekanntmachung des Hinweises auf die Patenterteilung: **06.05.1998**
(21) Anmeldenummer: 94104873.8
(22) Anmeldetag: 28.03.1994
(51) Int. Cl.: G01N 33/15, B30B 11/00

(54) **Verfahren und Vorrichtung zum automatischen Prüfen und Qualitätsbestimmen von Tabletten oder Pillen**
Method and apparatus for testing and determining the quality of tablets or pills automatically
Méthode et appareil pour tester et déterminer automatiquement la qualité de comprimés ou pilules

(30) Priorität: 29.03.1993 DE 4309978
(43) Veröffentlichungstag der Anmeldung: 05.10.1994
(73) Patentinhaber: Krämer, Norbert, 64291 Darmstadt (DE)
(72) Erfinder: Krämer, Norbert, 64291 Darmstadt (DE)
(74) Vertreter: Mierswa, Klaus, Dipl.-Ing.

(56) Entgegenhaltungen:
- DE-A- 3 628 757
- US-A- 4 452 579
- US-A- 4 537 229

## Beschreibung

### Technisches Gebiet:

Die Erfindung betrifft ein Verfahren zum automatischen Prüfen und Qualitätsbestimmen von aus einer Tabletten- oder Pillenpreßmaschine fortlaufend kommenden Tabletten oder Pillen sowie eine Vorrichtung hierzu.

### Stand der Technik:

Bei der Tabletten- oder Pillenherstellung werden die aus der Tablettenpreßmaschine kontinuierlich geförderten Tabletten normalerweis in Fässer gefüllt, wobei eine Charge von Fässern einen Batch darstellt. Aus dem Tablettenstrom aus der Tablettenpreßmaschine werden in bestimmten Zeitabständen Muster gezogen (Musterzüge), die einer Einrichtung zur automatischen Qualitätskontrolle zugeführt werden. In dieser Einrichtung werden die Muster verschiedenen Tests unterworfen, wie Härtetest, Dickenmessung, Durchmesser-bestimmung, Abwiegen, Kalibrierung u.a. Die Ergebnisse dieser Prüfungen werden in Protokollen zusammengefaßt. Die Herausnahme der Musterzüge aus dem Tablettenstrom erfolgt während des normalen Befüllvorgangs der Fässer, wobei die Fässer automatisch gefüllt und somit der Tablettenstrom von einem vollen Faß in ein leeres Faß geleitet werden kann. Derartige Arbeitsweisen sind u.a. durch die DE-41 18 878-A1 bekannt geworden.

Da die Entnahme der Musterzüge unabhängig vom Füllvorgang der Fässer erfolgt und diese auch während der Entnahme der Musterzüge weiterhin gefüllt werden, ist es bis heute nicht möglich, den von der Prüfeinrichtung erstellten Protokollen ein bestimmtes Faß zuzuordnen; bis heute ist nur die Zuordnung der Protokolle zu einer gesamten Charge möglich. Wenn somit innerhalb der Protokolle Ausreißer entdeckt werden, die aufgrund der vorgegebenen Qualitätsbestimmungen nicht mehr tragbar sind, so muß die gesamte Charge verworfen werden, was mit erheblichen Kosten verbunden ist.

Durch die DE-36 28 757-A1 ist ein Verfahren zur Qualitätssicherung bei der Herstellung von Tabletten bekannt geworden, bei dem während der Herstellung der Tabletten Proben gezogen werden, die aus einer Vielzahl von Tabletten bestehen, deren gemeinsamer Gewichts-Istwert mit dem Gewichts-Sollwert verglichen wird und bei dem die Tablettiermaschiene entsprechend der Abweichung zwischen Istwert und Sollwert nachgeregelt wird, um das Tablettengewicht auf den Sollwert zu bringen. Vor einer Nachregelung der Tablettiermaschiene werden die Gewichte der einzelnen Tabletten der Probe gemessen und miteinander verglichen. Bei Ermittlung einer oder mehrerer im Gewicht vom Sollwert wesentlich abweichender Tabletten der Proben wird die Statistik durch Korrekturrechnung berichtigt und der berichtigte Wert zur Nachregelung benutzt. Die statistische Auswertung der Meßergebnisse sowie eine automatische Korrekturberechnung oder Anforderung einer zweiten Probe erfolgt durch einen Rechner, dessen Datenausgang mit einer Einrichtung zur Nachregelung der Füllgutmenge verbunden ist. Dieses Verfahren wird bei der Herstellung der Tabletten eingesetzt und gestattet deshalb nicht eine Zuordnung der Meßergebnisse zu einem Faß, da, wie oben gesagt, die Entnahme eines Musterzuges unabhängig vom Füllvorgang der Fässer erfolgt.

### Technische Aufgabe:

Der Erfindung liegt die Aufgabe zugrunde, ein Verfahren und eine Vorrichtung der eingangs genannten Gattung zu schaffen, mit dem eine Zuordnung von aus einem Tablettenstrom entnommenen Mustertabletten zu einem bestimmten Behälter möglich ist, welcher gerade von der Tablettenpreßmaschine gefüllt wird.

### Offenbarung der Erfindung und deren Vorteile:

Die Lösung der Aufgabe besteht erfindungsgemäß in einem Verfahren zum automatischen Prüfen und Qualitätsbestimmen von aus einer Tabletten- oder Pillenpreßmaschine fortlaufend kommenden Tabletten oder Pillen, die in eine Mehrzahl von Behältern gefördert werden, wobei dem Tabletten- oder Pillenstrom für die Durchführung einer automatischen Qualitätskontrolle Tabletten- oder Pillenproben (Musterzug) entnommen werden, die innerhalb einer Tabletten- oder Pillenprüfeinrichtung nach vorgegebenen Qualitätsmerkmalen geprüft werden und ist dadurch gekennzeichnet daß a) die Behälter je auf einer Waage angeordnet sind, und das Gewicht des momentan befüllten Behälters samt Inhalt an Tabletten oder Pillen fortlaufend gemessen, in ein entsprechendes elektrisches Signal gewandelt und dieses fortlaufend einer Rechen- und Steuereinheit innerhalb der Tabletten- oder Pillenprüfeinrichtung aufgegeben wird, b) für jeden Behälter eine Identifizierung erfolgt, die ebenfalls der Rechen- und Steuereinheit aufgegeben wird, c) in vorgegebenen Intervallen aus dem Tabletten- oder Pillenstrom, der in den momentan zu befüllenden Behälter läuft, Tabletten- oder Pillenprüflinge entnommen und der Tabletten- oder Pillenprüfeinrichtung zugeführt und dort nach vorgegebenen Qualitätsmerkmalen geprüft werden, d) die Prüfergebnisse unter Zuordnung zum momentan zu befüllenden Behälter von der Rechen- und Steuereinheit gespeichert und gegebenenfalls auf einen Druckträger visuell sichtbar als Protokoll für den entsprechenden Behälter ausgedruckt werden. Eine Vorrichtung zur Durchführung des Verfahrens besteht in den Merkmalen des Anspruchs 6. Weitere vorteilhafte Ausgestaltungen der Erfindung sind in den übrigen Unteransprüchen gekennzeichnet.

Das erfindungsgemäße Verfahren besitzt den hervorstechenden Vorteil, daß mit diesem entweder gewichtsgesteuert oder zeitgesteuert Musterzüge dem Tablettenstrom entnommen, der Prüfeinrichtung zugeführt werden und anschließend die Protokolle eindeutig dem gerade gefüllten Behälter zugeordnet werden können.

Zur Erläuterung soll beispielsweise das Tablettengewicht eines fertig gefüllten Behälters 18kg betragen. Das Gewicht des Behälters vom leeren bis zum vollen Zustand wird als eketrisches Signal kontinuierlich über die Schnittstelle der Waage der Tabletten- oder Pillenprüfeinrichtung aufgegeben. In vorgegebenen Intervallen erfolgt z.B. gewichtsgesteuert, z.B. in 1-Kilogramm-Schritten, die laufende Entnahme von Musterzügen, so daß bei einem Endgewicht von 18 kg pro Behälter, 18 Musterzüge pro Behälter gezogen werden. Die Protokolle für diese Musterzüge können gemeinsam ausgedruckt werden, beispielsweise auf ein selbstklebendes Label, welches auf den entsprechenden Behälter geklebt werden kann.

Ebenso können die vorgegebenen Intervalle nur zeitgesteuert sein, daß z.B. alle 10 Minuten ein Musterzug gezogen wird. Bei einer Befüllzeit von beispielsweise 150 Minuten pro Behälter werden auf diese Weise 15 Musterzüge gezogen und überprüft. Wesentlich ist dabei, daß eine Identifizierung bzw. Markierung des betreffenden Behälters erfolgt, welcher sich gerade auf der Waage befindet und befüllt wird.

In vorteilhafter Weise geschieht die Anforderung der Musterzüge von der Tabletten- oder Prüfeinrichtung aus, die aus dem Tablettenstrom, der in einen bestimmten Behälter gefüllt wird, selbsttätig die Musterzüge entnimmt. Dazu fordert das Prüfsystem das aktuelle Gewicht des Behälters, die Behälternummer und auch den Behälterwechsel an, wobei der Behälterwechsel entweder manuell oder automatisch ausgeführt werden kann.

In vorteilhafter Weise stellt die Erfindung zum ersten Mal ein Verfahren und eine Vorrichtung zur Verfügung, die eine Kommunikation zwischen einer Waage mit elektronischer Schnittstelle und einem Prüfsystem dergestalt ermöglichen, daß die erstellten Protokolle mit den Prüfkriterien für eine Mehrzahl von Musterzügen direkt dem entsprechenden Behälter zugeordnet werden können, welcher gerade während der Entnahme der betreffenden Musterzüge befüllt wird bzw. gerade befüllt worden ist.

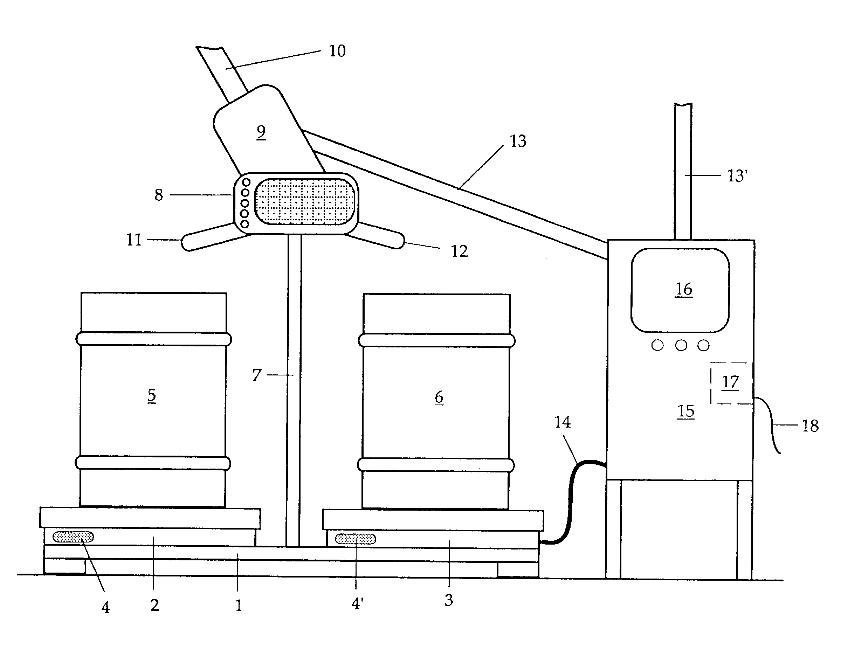

Die Zeichnung zeigt ein Beispiel einer Vorrichtung, mit der das erfindungsgemäße Verfahren durchgeführt werden kann.

### Bevorzugte Ausführung der Erfindung:

Auf einem Support 1 ist wenigstens eine Waage, im gezeigten Beispiel zwei Waagen 2, 3, angeordnet, wobei jede Waage 2,3 eine elektronische Kraftmeßdose 4, 4' und eine Schnittstelle aufweist und auf die Waage jeweils ein Behälter 5, 6 gestellt werden kann. Gleichzeitig kann der Support 1 eine Tragsäule 7 aufweisen, an der ein Bedienfeld 8 mit einer Eingabetastatur angeordnet ist.

Eine Weiche 9 weist zwei Auslaufrohre 11, 12 auf, die jeweils einem der Behälter 5, 6 auf den Waagen 2,3 zugeordnet sind; in die Weiche 9 führt eine Zuführleitung 10, die zu einer nichtgezeigten Tablettenpreßmaschine führt. Desweiteren kann die Weiche einen dritten Auslaß 13 aufweisen, der zu einer Tablettenprüfeinrichtung 15 führt, die eine Anzeigeeinrichtung 16 sowie einen Protokolldrucker 17 aufweist. Desweiteren kann die Prüfeinrichtung 15 eine direkte Zuführleitung 13' zur Tablettenpreßmaschine besitzen. Die Waagen 2, 3 bzw. die Kraftmeßdosen 4, 4' sind über die Schnittstellen und einer elektrischen Zuführleitung 14 mit der Prüfeinrichtung 15 verbunden.

Die Tablettenpreßmaschine erzeugt fortlaufend Tabletten, die über die Zuführleitung 10 der Weiche 9 zugeführt werden. Es sei angenommen, daß momentan der Behälter 6 befüllt wird. Nach dem Aufstellen des Behälters 6 auf die Waage 3 wird der Behälter über die Schnittstelle der Waage 3 von der Prüfeinrichtung 15 identifiziert, wobei die einfachste Identifizierung eine fortlaufende Numerierung der Behälter darstellt, die nacheinander gefüllt werden, wobei zur Identifikation der einzelnen Behälter nur die genaue Reihenaufgabe der Behälter 5, 6 auf die Waagen 2, 3 und die Abnahme der gefüllten Behälter in Reihe von den Waagen zu erfolgen hat. Zu Beginn des Befüllvorganges wird das Tara-Gewicht des Behälters der Prüfeinrichtung eingegeben. Desweiteren ist die Entnahme von Musterzügen über die Prüfeinrichtung 15 derart voreingestellt, daß beispielsweise während des Befüllvorganges pro n-Kilogramm Füllgewicht ein Musterzug entnommen wird. Dazu wird das aktuelle Gewicht des Behälters 6 auf die Prüfeinrichtung 15 aufgegeben, die jeweils nach jeder Vollendung eines n-kg-Schrittes einen Musterzug entweder aus der Weiche 9 über die Zuführleitung 13 oder direkt aus der Tablettenpreßmaschine über die Zuführleitung 13' anfordert. Wenn beispielsweise der Behälterinhalt 18 kg betragen soll, so sind am Ende des Befüllvorganges des Behälters bei 1-kg-Schritten 18 Musterzüge gezogen und überprüft worden. Sämtliche Protokolle der Musterzüge können nun mittels eines Druckers 17 auf einen Druckträger 18 ausgedruckt werden, wobei aufgrund der Identifizierung des betreffenden Behälters eine eindeutige Zuordnung zu dem jeweils gefüllten Behälter erfolgt. Beispielsweise kann der Druckträger 18 ein selbstklebendes Label sein, welches nach dem Füllvorgang und dem Erstellen sämtlicher Protokolle auf den betreffenden Behälter aufgeklebt wird. Nach dem Erreichen des Endgewichtes pro Behälter kann die Weiche 9 selbstätig auf den zwischenzeitlich herangeführten zweiten, leeren Behälter 5 umsteuern.

Wenn bei der Überprüfung der Protokolle festgestellt wird, daß in spezifischen Protokollen die vorgegebenen Qualitätsmerkmale der Tabletten oder Pillen nicht eingehalten worden sind, so muß nur der Inhalt des betreffenden Behälters ausgesondert werden, nicht jedoch die gesamte Charge, bestehend aus einer Mehrzahl von Behältern.

### Gewerbliche Anwendbarkeit:

Der Gegenstand der Erfindung ist insbesondere in der pharmazeutischen Industrie bei der Herstellung und Verarbeitung von Tabletten oder Pillen anwendbar, da mittels des erfindungsgemäßen Verfahrens bzw. der Vorrichtung entweder gewichtsgesteuert oder zeitgesteuert Musterzüge dem Tablettenstrom entnommen und der Prüfeinrichtung zugeführt werden und anschließend die Protokolle eindeutig dem gerade gefüllten bzw. dem in Befüllung befindlichen Behälter zugeordnet werden können.

## Patentansprüche

1. Verfahren zum automatischen Prüfen und Qualitätsbestimmen von aus einer Tabletten- oder Pillenpreßmaschine fortlaufend kommenden Tabletten oder Pillen, die in eine Mehrzahl von Behältern (5,6) gefördert werden, wobei dem Tabletten- oder Pillenstrom für die Durchführung einer automatischen Qualitätskontrolle Tabletten- oder Pillenproben (Musterzug) entnommen werden, die innerhalb einer Tabletten- oder Pillenprüfeinrichtung (15) nach vorgegebenen Qualitätsmerkmalen geprüft werden,
dadurch gekennzeichnet daß
a) die Behälter (5,6) je auf einer Waage (2,3) angeordnet sind, und das Gewicht des momentan befüllten Behälters samt Inhalt an Tabletten oder Pillen fortlaufend gemessen, in ein entsprechendes elektrisches Signal gewandelt und dieses fortlaufend einer Rechen- und Steuereinheit innerhalb der Tabletten- oder Pillenprüfeinrichtung (15) aufgegeben wird,
b) für jeden Behälter (5,6) eine Identifizierung erfolgt, die ebenfalls der Rechen- und Steuereinheit aufgegeben wird,
c) in vorgegebenen Intervallen aus dem Tabletten- oder Pillenstrom, der in den momentan zu befüllenden Behälter (5,6) läuft, Tabletten- oder Pillenprüflinge entnommen und der Tabletten- oder Pillenprüfeinrichtung (15) zugeführt und dort nach vorgegebenen Qualitätsmerkmalen geprüft werden,
d) die Prüfergebnisse unter Zuordnung zum momentan zu befüllenden Behälter (5,6) von der Rechen- und Steuereinheit gespeichert und gegebenenfalls auf einen Druckträger visuell sichtbar als Protokoll für den entsprechenden Behälter ausgedruckt werden.

2. Verfahren nach Anspruch 1,
dadurch gekennzeichnet,
daß die vorgegebenen Intervalle zur Entnahme der Musterzüge in Stufen gemäß dem zunehmenden Gewicht des Behälters (5,6) während des Befüllens gewichtsgesteuert erfolgt, zum Beispiel in n-kg-Schritten, so daß pro nkg Gewichtszunahme des Behälters die Entnahme eines Musterzuges erfolgt.

3. Verfahren nach Anspruch 1 oder 2,
dadurch gekennzeichnet,
daß die Entnahme der Musterzüge zeitgesteuert, zum Beispiel in n-Minuten-Schritten erfolgt, so daß pro nMinuten die Entnahme eines Musterzuges erfolgt.

4. Verfahren nach einem der vorherigen Ansprüche,
dadurch gekennzeichnet,
daß am Ende des Befüllvorgangs die Protokolle sämtlicher Musterzüge für einen bestimmten Behälter zusammengefaßt und ausgedruckt werden.

5. Verfahren nach einem der vorherigen Ansprüche,
dadurch gekennzeichnet,
daß die Rechen- und Steuereinheit der Tabletten- oder Pillenprüfeinrichtung (15) fortlaufend das aktuelle Gewicht der Behälter (5,6), für die Identifizierung eine Behälternummer und einen Behälterwechsel sowie die Musterzüge anfordert.

6. Vorrichtung zum automatischen Prüfen und Qualitätsbestimmen von aus einer Tabletten- oder Pillenpreßmaschine fortlaufend kommenden Tabletten oder Pillen, und zum Fördern in eine Mehrzahl von Behältern (5,6), wobei dem Tabletten- oder Pillenstrom für die Durchführung einer automatischen Qualitätskontrolle Tabletten- oder Pillenproben (Musterzug) entnehmbar sind, die innerhalb einer Tabletten- oder Pillenprüfeinrichtung (15) nach vorgegebenen Qualitätsmerkmalen geprüft werden, gekennzeichnet durch
a) eine eine elektronische Schnittstelle aufweisende Waage (2,3), auf der jeweils ein Behälter (5,6) angeordnet ist, die das Gewicht des momentan befüllten Behälters samt dem Inhalt an Tabletten oder Pillen fortlaufend zu messen imstande ist und die ein entsprechendes elektrisches Signal abgibt,
b) eine Identifizierungseinrichtung des Behälters (5,6),
c) eine der Tabletten- oder Pillenprüfeinrichtung (15) zugeordnete elektronische Rechen- und Steuereinheit, auf die über die Schnittstelle das aktuelle elektrische Signal der Waage sowie die Identifizierung des jeweiligen Behälters (5,6) aufgegeben sind, wobei dem Tabletten- oder Pillenstrom, der in den momentan zu befüllenden Behälter läuft, in vorgegebenen Intervallen Tabletten- oder Pillenprüflinge entnommen und der Tabletten- oder Pillenprüfeinrichtung zugeführt und geprüft werden,
d) eine Speicher- und Ausgabeeinheit (16,17) innerhalb der Tabletten- oder Pillenprüfeinrichtung (15) zum Speichern und Ausgeben der Prüfergebnisse unter Zuordnung zum momentan zu befüllenden Behälter (5,6), wobei die Prüfergebnisse sämtlicher Musterzüge für einen Behälter zusammengefaßt sind.

7. Vorrichtung nach Anspruch 6,
dadurch gekennzeichnet,
daß die vorgegebenen Intervalle zur Entnahme der Musterzüge in Stufen gemäß dem zunehmenden Gewicht des Behälters (5,6) während des Befüllens gewichtsgesteuert oder in zeitlichen Intervallen nur zeitgesteuert erfolgt, zum Beispiel in n-kg-Schritten bzw. in n-Minuten-Schritten.

## Claims

1. Process for automatically testing and determining the quality of tablets or pills which are continuously being discharged from a tablet or pill pressing machine and which are conveyed into a plurality of containers (5, 6), whereby, in order to carry out automatic quality control, tablet or pill samples are taken from the tablet or pill stream (sampling) and are then tested in a tablet or pill testing device (15) according to prescribed quality features,
characterized in that
a) the containers (5, 6) are each positioned on a set of scales (2, 3), and the weight of the container currently being filled, including its content of tablets or pills, is continuously measured, converted into a corresponding electric signal, and continuously transmitted to a computer and control unit located in the tablet or pill testing device (15),
b) for each container (5, 6), identification is carried out, which is likewise transmitted to the computer and control unit,
c) at prescribed intervals, tablet or pill test samples are taken from the tablet or pill stream which is flowing into the container (5, 6) currently being filled and are conveyed to the tablet or pill testing device (15) where they are then tested according to prescribed quality features,
d) the test results are associated with the container (5, 6) currently being filled and stored by the computer and control unit, and optionally printed visually on a printout that serves as a record for the corresponding container.

2. Process according to Claim 1,
characterized in that
the prescribed intervals for taking the samples are weight-controlled in increments as a function of the increasing weight of the container (5, 6) during the filling operation, for example, in n-kg increments, so that one sample is taken every time the weight of the container increases by n kg.

3. Process according to Claim 1 or 2,
characterized in that
the taking of the samples is time-controlled, for example, in n-minute increments, so that one sample is taken every n minutes.

4. Process according to one of the preceding Claims,
characterized in that,
at the end of the filling procedure, the records of all of the samples taken for a given container are compiled and printed out.

5. Process according to one of the preceding Claims,
characterized in that
the computer and control unit of the tablet and pill testing device (15) continuously requests the current weight of the containers (5, 6), a container number for the identification and a container change as well as the samples taken.

6. Device for automatically testing and determining the quality of tablets or pills which are continuously being discharged from a tablet or pill pressing machine and for conveying them into a plurality of containers (5, 6), whereby, in order to carry out automatic quality control, tablet or pill samples can be taken from the tablet or pill stream (sampling) and are then tested in a tablet or pill testing device (15) according to prescribed quality features,
characterized by
a) a set of scales (2, 3) which has an electronic interface and on which a container (5, 6) is positioned, whereby said set of scales can continuously measure the weight of the container currently being filled, including its content of tablets or pills, and transmits a corresponding electric signal,
b) an identification means of the container (5, 6),
c) an electronic computer and control unit which is associated with the tablet or pill testing device (15) and to which the current electric signal from the scales as well as the identification of the corresponding container (5, 6) are transmitted, whereby, at prescribed intervals, tablets or pill samples are taken from the tablet or pill stream which is flowing into the container currently being filled and transported to the tablet or pill testing device and then tested,
d) a storage and output unit (16, 17) located in the tablet or pill testing device (15) for the storage and output of the test results, associated with the container (5, 6) currently being filled, whereby the test results of all of the samples taken for a given container are compiled.

7. Device according to Claim 6,
characterized in that
the prescribed intervals for taking the samples are weight-controlled in increments as a function of the increasing weight of the container (5, 6) during the filling, for example, in n-kg increments, or else exclusively time-controlled in time intervals, for example, in n-minute increments.

## Revendications

1. Méthode pour tester et déterminer automatiquement la qualité de comprimés ou de pilules provenant en continu de presses à comprimés et transportés dans plusieurs fûts (5,6), les échantillons de comprimés ou de pilules (prise de spécimen) étant prélevés du flux de comprimés ou de pilules pour pouvoir pratiquer un contrôle de qualité automatique et étant testés dans un appareil de contrôle de comprimés ou pilules (15) en fonction de critères de qualité définis,
caractérisée en ce que
a) les fûts (5,6) sont chacun placés sur une balance (2,3) et le poids du fût en cours de remplissage est mesuré en continu avec son contenu en comprimés ou pilules, et transformé en un signal électrique correspondant qui est transmis en continu à une unité de calcul et de commande à l'intérieur de l'appareil de contrôle de comprimés ou pilules (15),
b) pour chacun des fûts (5,6) a lieu une identification qui est également communiquée à l'unité de calcul et de commande,
c) des échantillons de comprimés ou de pilules sont prélevés à des intervalles prédéterminés du flux de comprimés ou de pilules qui est déversé dans le fût (5,6) en cours de remplissage, et sont amenés dans l'appareil de contrôle de comprimés ou pilules (15) dans lequel ils sont testés en fonction de critères de qualité définis,
d) les résultats des tests de contrôle sont mémorisés par l'unité de calcul et de commande, tout en étant assignés au fût en cours de remplissage (5,6) auquel ils sont assortis, et sont, si besoin est, visualisés sous forme de protocole sur un support d'impression sur lequel ils sont édités pour le fût auquel ils sont assignés.

2. Méthode selon la revendication 1,
caractérisée en ce que
les intervalles prédéterminés pour le prélèvement des échantillons sont asservis au poids et échelonnés par paliers au fur et à mesure que le poids du fût (5,6) augmente pendant le remplissage, par exemple par paliers de n kg, de sorte qu'une prise de spécimen a lieu par prise de poids de n kg du fût.

3. Méthode selon l'une des revendications 1 ou 2,
caractérisée en ce que
le prélèvement des échantillons est asservi au temps et est échelonné, par exemple, en plages de n minutes, de sorte que toutes les n minutes a lieu une prise de spécimen.

4. Méthode selon l'une des revendications ci-avant,
caractérisée en ce que
le processus de remplissage une fois terminé, les protocoles relatifs à toutes les prises de spécimen se rapportant à un même fût sont regroupés puis édités.

5. Méthode selon l'une des revendications ci-avant,
caractérisée en ce que
l'unité de calcul et de commande de l'appareil de contrôle de comprimés ou pilules (15) se renseigne de façon suivie sur le poids actuel du fût (5,6), le numéro de fût pour l'identification et le changement de fût, ainsi que sur les prises de spécimen.

6. Dispositif destiné à tester et déterminer automatiquement la qualité de comprimés ou de pilules provenant en continu d'une presse à comprimés et à les transporter dans plusieurs fûts (5,6), des échantillons de comprimés ou de pilules (prise de spécimen) pouvant être prélevés du flux de comprimés ou de pilules pour pratiquer un contrôle de qualité automatique et qui sont testés dans un appareil de contrôle pour comprimés ou pilules (15) en fonction de critères de qualité définis,
caractérisé par
a) une balance (2,3), dotée d'une interface électronique, sur laquelle est placé un fût (5,6), et qui est à même de mesurer en continu le poids du fût en cours de remplissage et de son contenu en comprimés ou pilules, et qui émet un signal électrique correspondant,
b) un dispositif d'identification du fût (5,6)
c) une unité de calcul et de commande électronique assignée à l'appareil de contrôle de comprimés ou pilules (15) à laquelle sont communiqués via une interface le signal électrique actuel ainsi que l'identification du fût correspondant (5,6), des échantillons de comprimés ou de pilules étant prélevés à des intervalles prédéterminés à partir du flux de comprimés ou de pilules qui est déversé dans le fût en cours de remplissage, puis guidés et testés dans l'appareil de contrôle de comprimés ou pilules
d) une unité mémoire et unité de sortie (16,17) à l'intérieur de l'appareil de contrôle de comprimés ou pilules (15) pour la mémorisation et l'édition des résultats des tests de contrôle, résultats assortis au fût en cours de remplissage (5,6), les résultats des tests de contrôle de toutes les prises de spécimen étant regroupés par fût.

7. Appareil selon la revendication 6,
caractérisé en ce que
les intervalles prédéterminés pour le prélèvement des échantillons sont asservis au poids et échelonnés par paliers au fur et à mesure que le poids du fût (5,6) augmente pendant le remplissage, ou sont des intervalles dans le temps uniquement asservis au temps, par exemple des paliers de n kg ou des plages de n minutes.
